# EUROPEAN PATENT APPLICATION

(11) **EP 0 730 844 A1**
(43) Date of publication of application: **11.09.1996**
(21) Application number: 95120722.4
(22) Date of filing: 29.12.1995
(51) Int. Cl.: A61B 5/05, H01R 13/719, H01R 13/658

(54) **Electronic endoscope apparatus**

(30) Priority: 22.03.1995 JP 62890/95; 20.03.1995 JP 61049/95; 10.03.1995 JP 51153/95
(71) Applicant: OLYMPUS OPTICAL CO., LTD., Tokyo 151 (JP)
(72) Inventor: Takamura, Koji, Hachioji-shi, Tokyo (JP); Kishi, Takahiro, Machida-shi, Tokyo (JP); Yabe, Hisao, Hachiojo-shi, Tokyo (JP); Yamaya, Koji, Hachiojo-shi, Tokyo (JP); Nakazawa, Masaaki, Hino-shi, Tokyo (JP); Ito, Hideo, Akishima-shi, Tokyo (JP); Ishii, Hiroshi, Hachioji-shi, Tokyo (JP)
(74) Representative: Kahler, Kurt, Dipl.-Ing.

(57) **Abstract**

An electronic endoscope apparatus (1) according to the invention is provided with an electronic endoscope (2) having arranged thereon a solid-state image pickup element as image pickup means at an insertion part (10), and external devices such as a light source device (3) for supplying an illumination light to the electronic endoscope (2), a video processor (4) for processing to a video signal an electric signal transmitted from the solid-state image pickup element of the electronic endoscope (2), and the like. An electric connection part (14) for electrically connecting the electronic endoscope (2) and the video processor (4) to each other is provided at a connector part (13) which is provided at an end of a universal cord (12) which connects the electronic endoscope (2) and the light source device (3) to each other. An electromagnetic interference countermeasure member is provided which covers at least one of the connector part (13), the universal cord (12) and the external devices (3, 4).

## Description

### BACKGROUND OF THE INVENTION

### Field of the Invention

The present invention relates to an electronic endoscope apparatus for reducing radiation of unnecessary radiative noises from an electronic endoscope and mixture or contamination of the unnecessary radiative noises to the electronic endoscope.

### Related Art Statement

Generally, the electronic endoscope is arranged as follows. That is, a solid-state image pickup element is arranged at a forward end of the electronic endoscope. An image pickup cable extends through a circuit substrate and electronic parts which are connected to the solid-state image pickup element. The image pickup cable is adapted to be inserted into and to pass through the interior of an insertion part, an operation part and a universal cord of the endoscope, and is connected to a connector part which is provided at an end of the universal cord.

Further, the electronic endoscope is connected to a processor, a light source device, a monitor and the like and is used as the electronic endoscope apparatus. Thus, by a plurality of operation switches which are arranged at the operation part of the electronic endoscope apparatus, it is possible to make an image stationary, and changeover of light modulation and control of external equipments. Within a universal cord, switch cables which extend from the operation switches are inserted and pass therethrough and are connected to the connector part.

On one hand, in recent years, also with respect to electric equipment devices or the like which are used within a hospital or the like, it has been desired more and more that EMC (which is a general name of EMI that is a problem which imparts electromagnetic disturbance or interference and EMS that is a problem which receives or is subjected to electromagnetic interference) countermeasures are sufficiently conducted. For this reason, also with respect to the above-described electronic endoscope apparatus, the EMC countermeasures have been mentioned as one of technical important matters.

Accordingly, in order to prevent that an electric signal which is used in the electronic endoscope apparatus is radiated to the outside of the apparatus to cause malfunction of other electronic equipments, or that noises which are radiated from other electronic equipments which reside outside are mixed within the electronic endoscope apparatus to reduce the image quality of an endoscope image, and to become a cause of malfunction of a control signal, the connector part which is provided with many electric contacts of the electronic endoscope apparatus is a part which particularly requires shielding strengthening.

For this reason, an arrangement in which a mechanism is provided which shields the connector part by a metal member, and an arrangement whose construction is such that an electromagnetic absorber member is provided between the electronic endoscope and a patient circuit are disclosed in Japanese Patent Unexamined Publication No. HEI 4-183432 (183432/1992). Moreover, disclosed in Japanese Patent Unexamined Publication No. HEI 6-142039 (142039/1994) is an arrangement in which a metal blade is provided within the universal cord, which has one end thereof conducted to a connector receipt, to thereby form electromagnetic screening or shielding means or electromagnetic interference countermeasure means.

However, there is the following problem. That is, for the construction in which the mechanism is provided to shield the connector part by the metal member as described above, for the construction in which the electromagnetic absorber member is provided between the endoscope and the patient circuit as disclosed in Japanese Patent Unexamined Publication No. HEI 4-183432, or the like, the connector part is large-sized and is weighed over so that convenience in use is deteriorated.

Furthermore, in order to incorporate the electromagnetic interference countermeasure means into the universal cord, there is a fear that not only much steps or man hours are required, but also convenience in use is deteriorated. If the electromagnetic interference countermeasure means is once incorporated into the electronic endoscope, an electronic endoscope in which the electromagnetic interference countermeasure has been ended exists regardless of whether or not the EMC countermeasures are necessary. For this reason, even also in a case where there is no interference if the conventional or prior art electronic endoscope is used, an electronic endoscope which has been ended in the electromagnetic interference countermeasure must be used, and a chance of selection is not given to a user. For this reason, an arrangement has been desired in which the EMC countermeasures can simply be conducted or performed to the existing electronic endoscope, as occasion demands, when the existing electronic endoscope is used in combination with, for example, an equipment which is apt to generate the noises.

### OBJECTS AND SUMMARY OF THE INVENTION

An object of the invention is to provide an electronic endoscope apparauts in which EMC countermeasures which reduce radiation and mixture of unnecessary radiative noises are conducted, which is small in size, which is light in weight and which is superior in convenience in use.

An another object of the invention is to provide an electronic endoscope apparauts in which the use under a state in which radiation and mixture of unnecessary radiative noises can sufficiently be reduced can be selected in accordance with the status or circumstances.

The other object of the invention is to provide an electronic endoscope apparauts in which EMC countermeasures can be formed easily and at low cost.

Briefly, an electronic endoscope apparatus according to the invention is provided with an electronic endoscope in which a solid-state image pickup element serving as image pickup means is arranged at an insertion part, and external devices such as a light source device for supplying an illumination light to the electronic endoscope, a video processor for processing an electric signal which is transmitted from the solid-state image pickup element of the electronic endoscope, to a video signal, and the like, in which the electronic endoscope apparatus is so arranged as to be provided with an electric connection part electrically connecting the electronic endoscope and the video processor to each other, at a connector part which is provided at an end of a universal cord which connects the electronic endoscope and the light source device to each other, and in which at least one of the connector part, the universal cord and the external devices is covered with an electromagnetic interference countermeasure member.

### BRIEF DESCRIPTION OF THE DRAWINGS

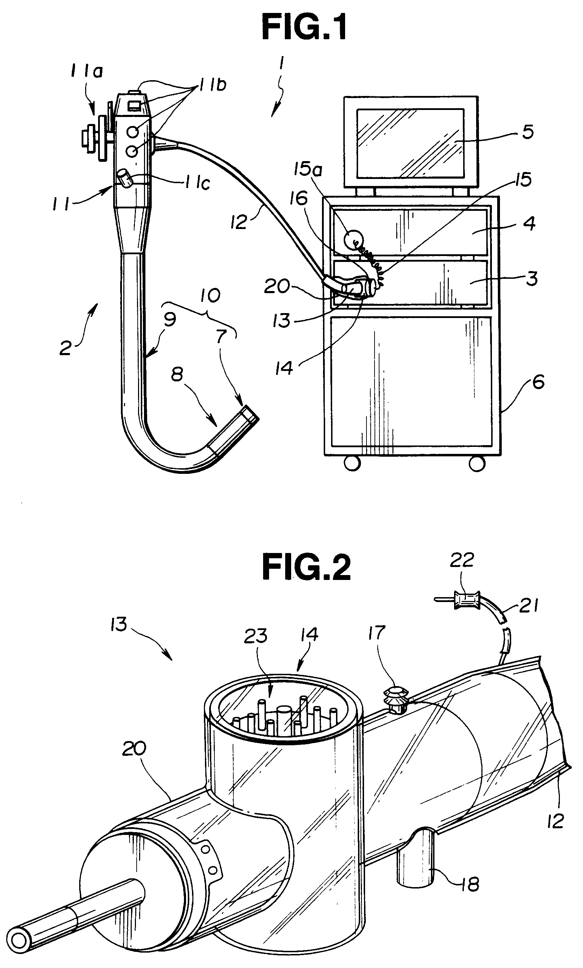

Figs. 1 and 2 are views describing a first embodiment according to the invention;
   Fig. 1 is an explanatory view showing a schematic arrangement of an electronic endoscope apparatus;
   Fig. 2 is a perspective view describing a connector part which has covered thereon an electromagnetic shielding cover as an electromagnetic interference countermeasure member;
Figs. 3 and 4 are explanatory views describing a second embodiment according to the invention;

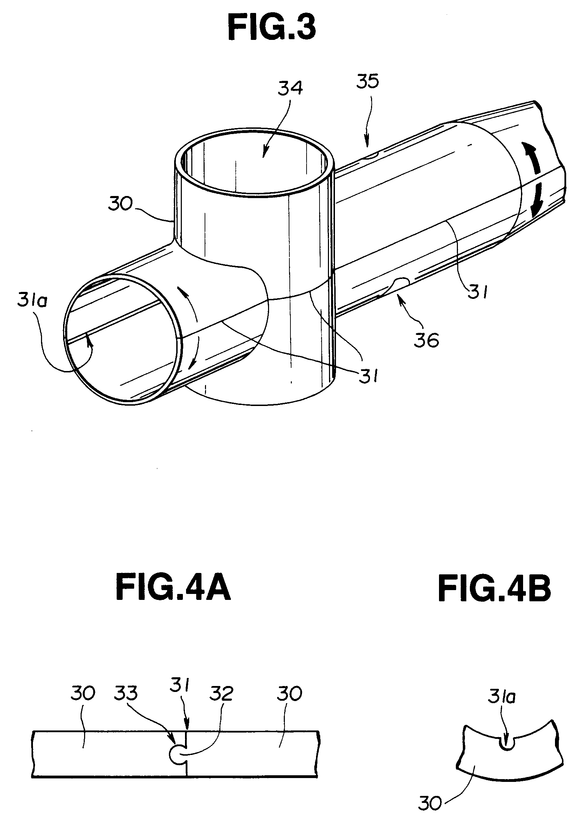

   Fig. 3 is a view showing an another arrangement of the electromagnetic shielding cover; and
   Fig. 4 is a developable electromagnetic shielding cover;
      Fig. 4A is a view showing an engagement state of a developable part of the electromagnetic shielding cover;
      Fig. 4B is a view describing an arrangement of the developable part of the electromagnetic shielding cover;

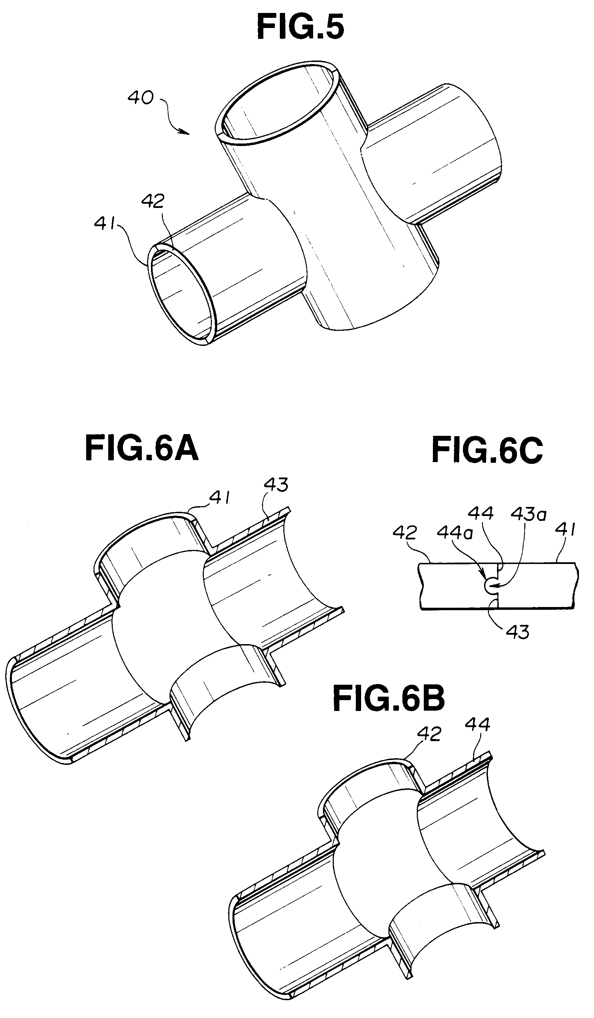

Figs. 5 and 6 are views describing a third embodiment according to the invention;
   Fig. 5 is a perspective view showing an another arrangement of the electromagnetic shielding cover;
   Fig. 6 is a view describing an arrangement of the electromagnetic shielding cover;
      Fig. 6A is a view showing a first shielding cover of the electromagnetic shielding cover;
      Fig. 6B is a view showing a second shielding cover of the electromagnetic shielding cover;
      Fig. 6C is a view showing an engagement state between the first shielding cover and the second shielding cover;

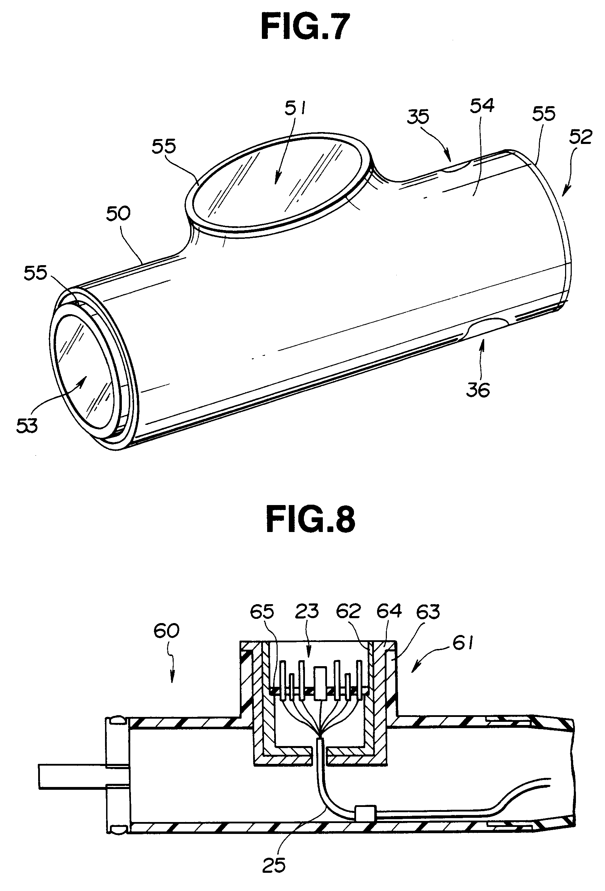

Fig. 7 is a view describing a fourth embodiment according to the invention and is a perspective view showing a still another arrangement of the electromagnetic shielding cover;
Fig. 8 is a cross-sectional view describing a connector part in which a radiative-noise reduction member is arranged at an electric connection part according to a fifth embodiment of the invention;

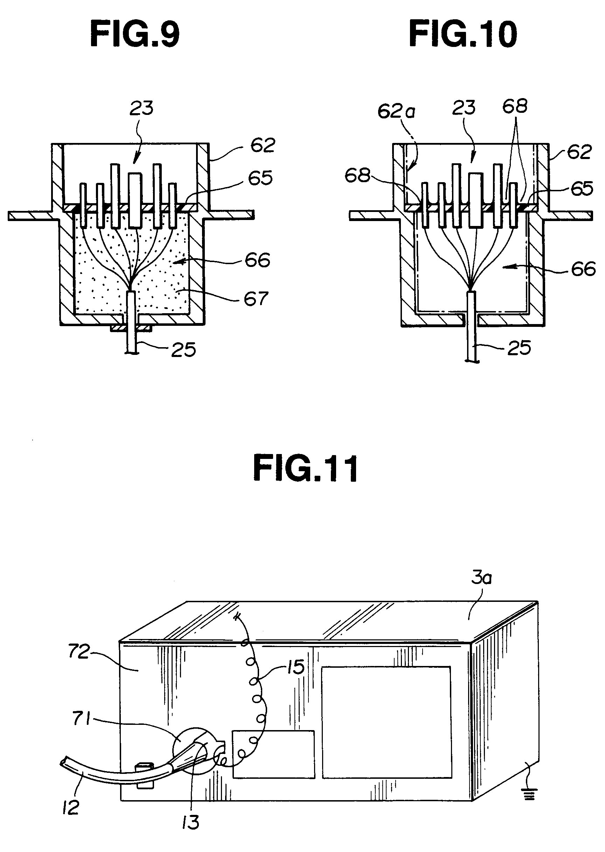

Fig. 9 is a cross-sectional view describing an arrangement in which an another radiative-noise reduction member is arranged at an electric connection part according to a sixth embodiment of the invention;
Fig. 10 is a cross-sectional view describing an another arrangement of the electric connection part in which a radiative-noise reduction member is arranged;
Fig. 11 is a perspective view describing a state in which a connector part is covered with a metal surrounding or enclosure member that is an electromagnetic interference countermeasure member relating to a seventh embodiment of the invention;

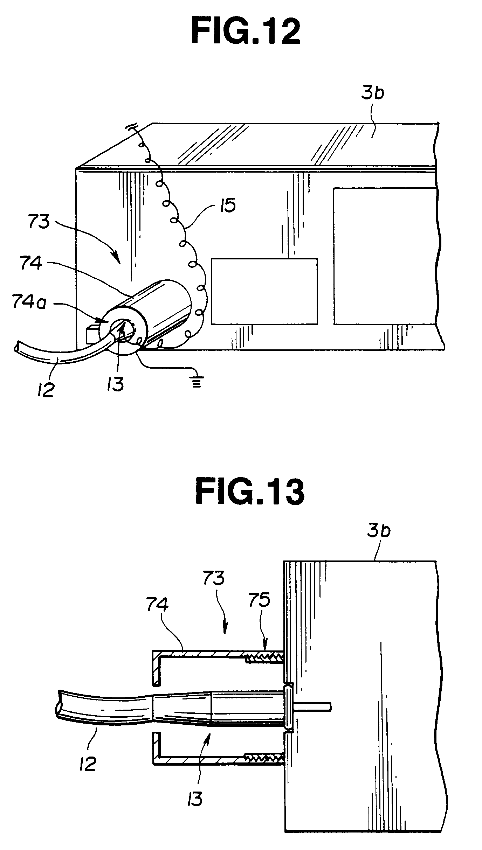

Fig.12 is a cross-sectional view describing an another arrangement of the metal enclosure member;
Fig. 13 is a cross-sectional view describing the metal enclosure member which is detachable with respect a light source device;

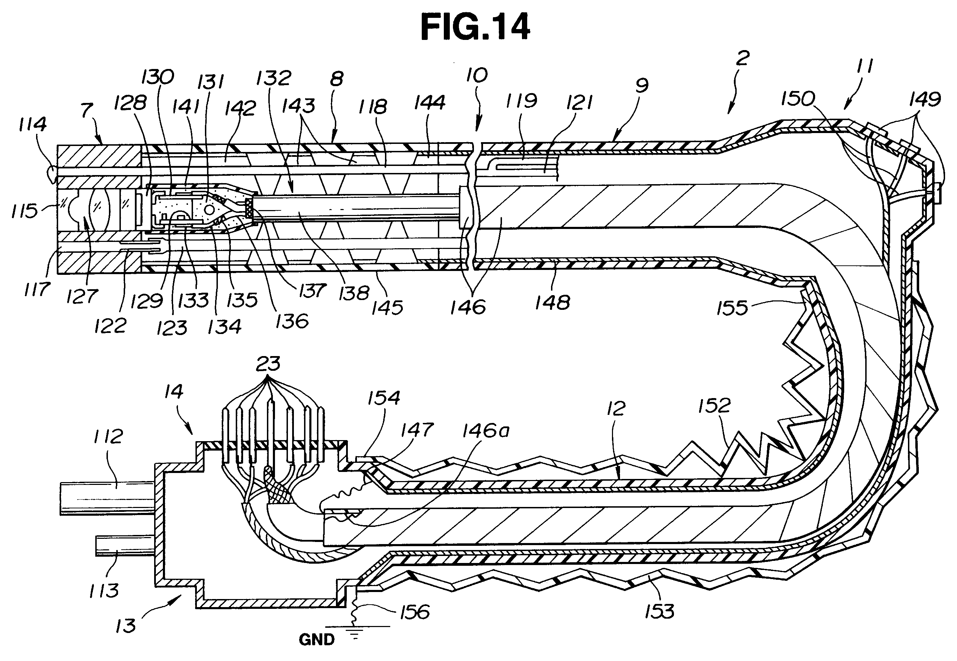

Figs. 14 to 16 are views describing an eighth embodiment according to the invention;
   Fig. 14 is a cross-sectional view describing an arrangement of an electronic endoscope;

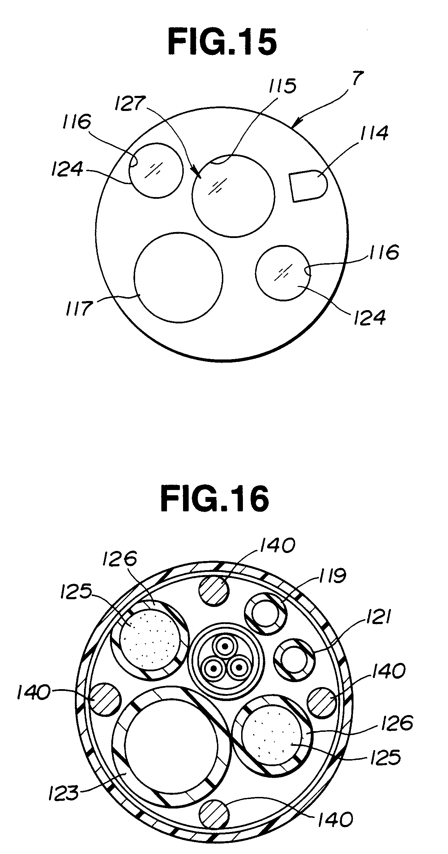

   Fig. 15 is a front elevational view as viewing the electronic endoscope from a forward end thereof;
   Fig. 16 is a cross-sectional view describing a structure of a flexible pipe part;

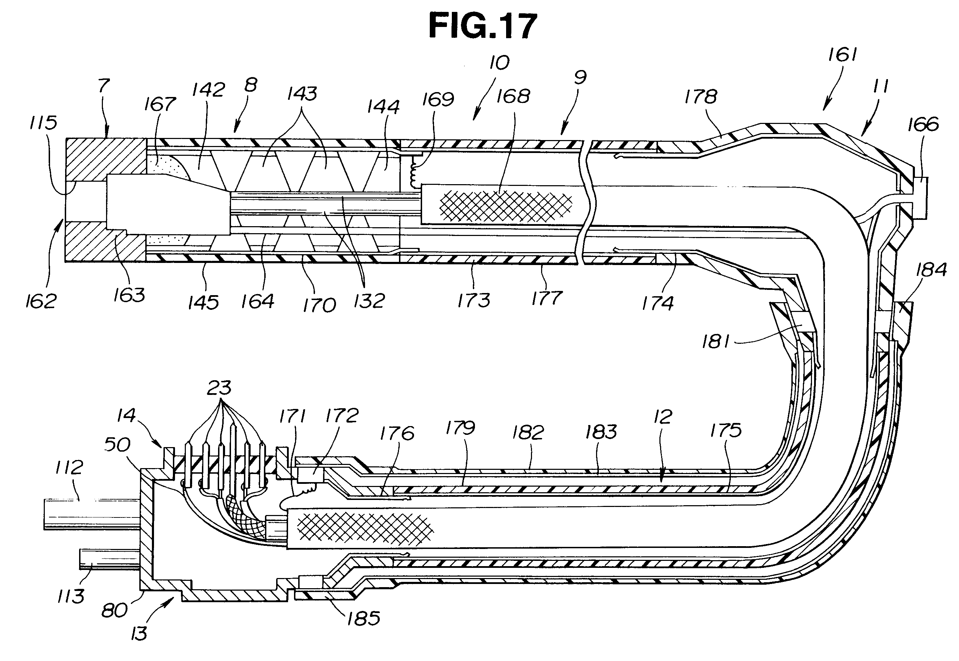

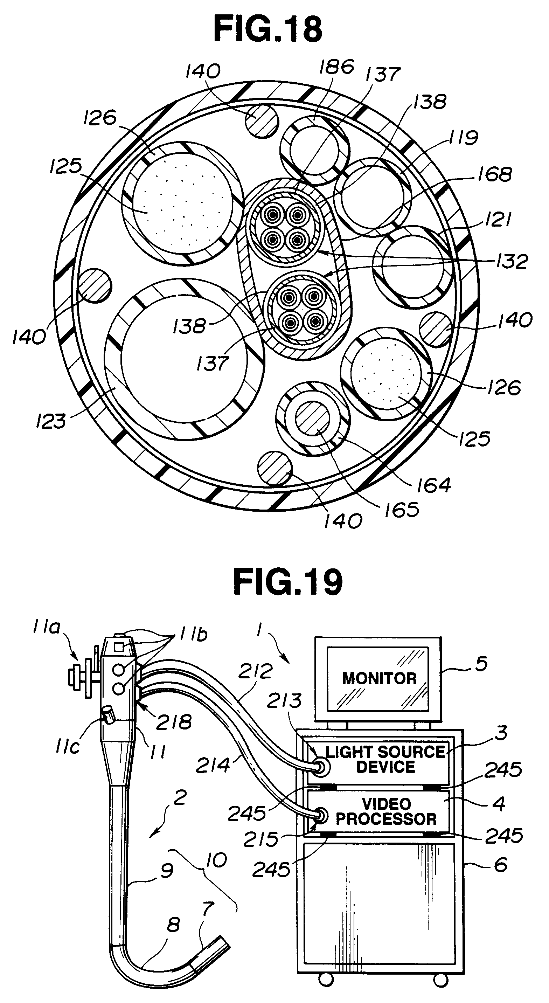

Figs. 17 and 18 are views describing a ninth embodiment according to the invention;
   Fig. 17 is a cross-sectional view describing an arrangement of an electronic endoscope;
   Fig. 18 is a cross-sectional view describing a structure of a flexible pipe part;
Figs. 19 to 24 are views describing a tenth embodiment according to the invention;
   Fig. 19 is a view showing an arrangement of an electronic endoscope apparatus;

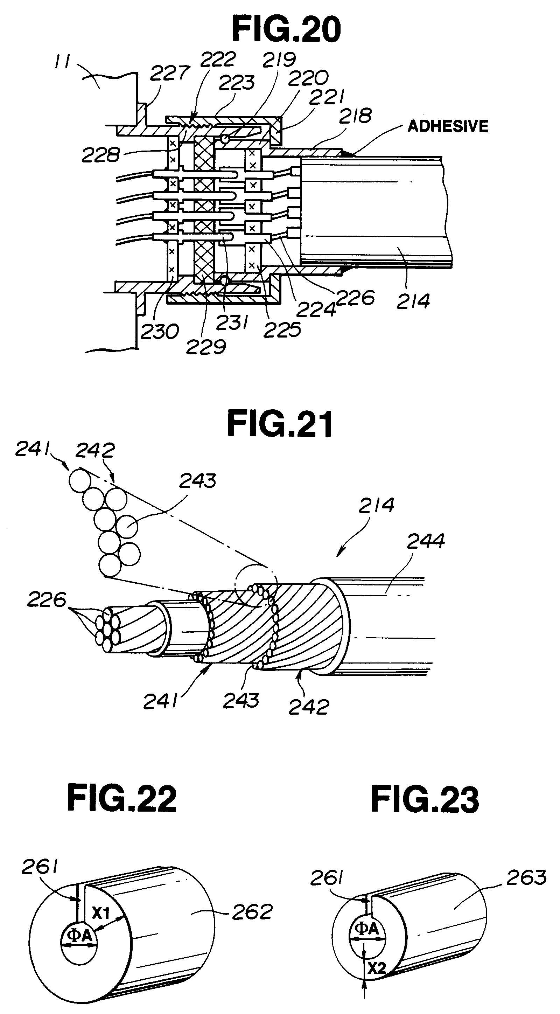

   Fig. 20 is an explanatory view showing a connection part between a signal cable and an operation part;
   Fig. 21 is a perspective view showing an internal arrangement of the signal cable;
   Fig. 22 is a perspective view showing an arrangement of a first electromagnetic absorber which is mounted to the signal cable;
   Fig. 23 is a perspective view showing an arrangement of a second electromagnetic absorber which is mounted to the signal cable;

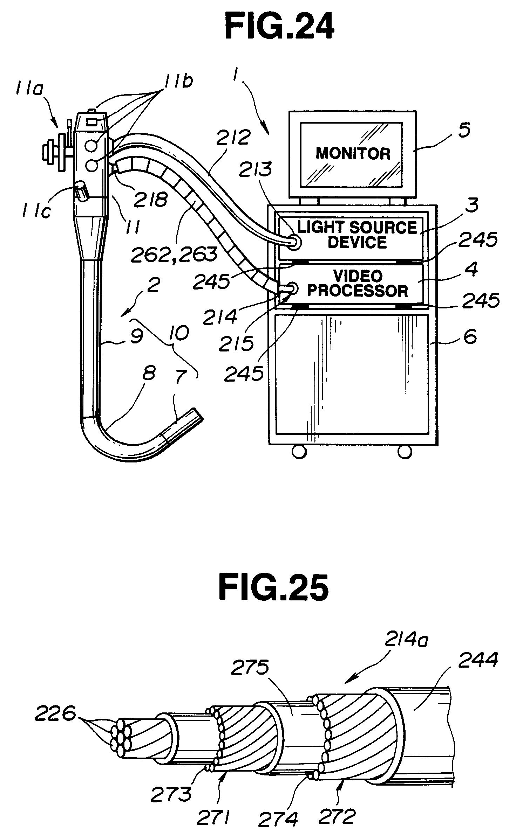

   Fig. 24 is a view showing the entirety or whole of the electronic endoscope apparatus which has the signal cable to which the electromagnetic absorbers are mounted;

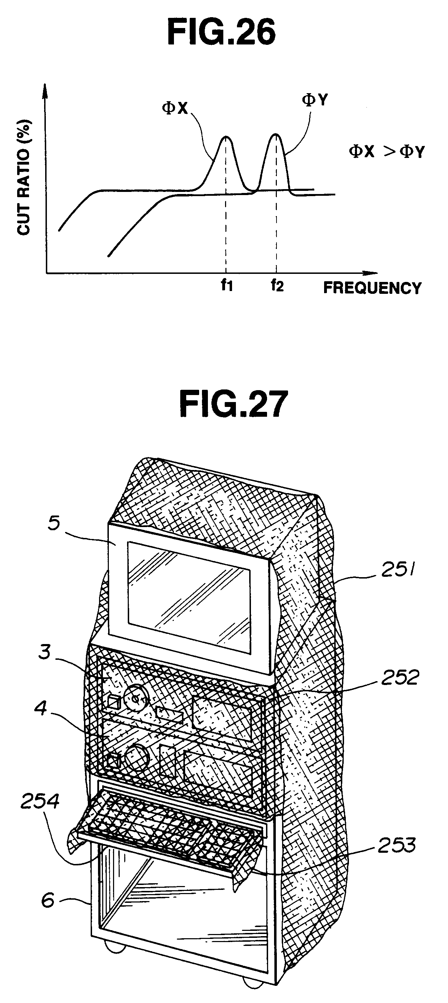

Figs. 25 and 26 are views describing an eleventh embodiment according to the invention; and
   Fig. 25 is a perspective view showing an internal arrangement of a signal cable;
   Fig. 26 is a characteristic view describing characteristics between a diameter size of the signal cable thereof and frequency;
Fig. 27 is a perspective view describing a state in which an external device relating to a twelfth embodiment of the invention and a cart on which the external device is mounted are covered with a shielding curtain that is an electromagnetic interference countermeasure member.

### DETAILED DESCRIPTION OF THE PREFERRED EMBODIMENTS

Various embodiments according to the invention will hereunder be described with reference to the accompanying drawings.

First, electromagnetic interference countermeasures of a connector part that is a part in which shielding strengthening or reinforcement is particularly required will be described

A first embodiment of the invention will be described with reference to Figs. 1 and 2.

As shown in Fig. 1, an electronic endoscope apparatus 1 principally comprises an electronic endoscope 2 having built therein a solid-state image pickup element to be described later such as a CCD or the like, and a cart 6 which stores therein a light source device 3 and a video processor 4 and which has an upper face part thereof on which a television monitor 5 is rested, at the side of a forward end of an insertion part.

The electronic endoscope 2 connects a forward-end forming part 7 having an image pickup part on which a solid-state image pickup element is arranged, a curvature part 8 having a plurality of curvature pieces and an elastic pipe part 9 having elasticity, to each other, from the forward-end side thereof, to form an elongated insertion part 10. The insertion part 10 has an end part thereof on the side of hand thereof to which an operation part 11 is connected on which a curvature operation knob 11a for curvedly operating the curvature part 8, buttons 11b for indicating gas supply, water supply and the like, an insertion port 11c through which a treatment tool is inserted into an unshown treatment-tool channel within the electronic endoscope 2, and the like. From the side of the operation part 11, a universal cord 12 extends through which a gas supply pipe, a water supply pipe, a suction pipe, a light guide, a plurality of signal cables and the like which are built in the electronic endoscope 2 are inserted and pass. The universal cord 12 has a distal end thereof on which a connector part 13 which is detachable to the light source device 3 is provided. The connector part 13 has an armor member thereof which is formed by thermoplastic resin such as polysulfone, denaturated PPO, polyetherimide or the like, principally for the purpose of reducing the weight thereof.

As shown in Fig. 2, an electric connector part 14 is provided on the side of the connector part 13. To the electric connector part 14, an electric-cord connector 16 of an electric cord 15 is detachably connected which is provided with a connector 15a which is detachably mounted to the video processor 4.

An electromagnetic shielding cover 20 which covers the whole of the connector part is covered on the connector part 13. The electromagnetic shielding cover 20 is a resin sheet which is formed by polyester, polyurethane or the like containing an electromagnetic absorber such as, for example, ferrite or the like, or an arrangement in which a metal thin wire such as stainless steel or the like is so wound as to be formed into a sheet-like form.

In a case, for example, where the use is made under a state in which the connector part 13 is covered with the electromagnetic shielding cover 20 in which the metal thin wire is wound so as to be formed into a sheet-like form, a terminal 22 which is provided at a forward end of a ground wire or line 21 which is electrically mounted on the electromagnetic shielding cover 20 by solder or the like is connected to a reference electric potential of the light source device 3, or is grounded through the light source device 3.

In connection with the above, in a case where a resin sheet containing the electromagnetic absorber is used for the electromagnetic shielding cover 20, it is unnecessary to provide the ground line 21 and the terminal 22. Moreover, the reference numeral 17 in Fig. 2 is a suction base. The reference numeral 18 is a gas-supply·water-supply base, and the reference numeral 23 is a group of contact pins which are connected to the electric cord 15.

In this manner, the connector part is covered with the resin sheet containing the electromagnetic absorber such as ferrite or the like, or with the electromagnetic shielding cover in the form of the sheet in which the metal thin wire such as stainless steel or the like is wound, whereby it is possible to provide the electronic endoscope apparatus in which the EMC countermeasures capable of executing the electromagnetic shielding which reduces radiation and mixture of the radiative noises are conducted without large-sizing and weighing-over of the connector part, regardless of a material and a construction which form the connector part.

A second embodiment of the invention will be described with reference to Figs. 3 and 4.

As shown in Fig. 3, an electromagnetic shielding cover 30 in the present enforcement form is formed into a developable thin plate form, in contrast to the fact that the electromagnetic shielding cover 20 in the first enforcement form is in the form of a sheet. Specifically, the electromagnetic shielding cover 30 is a cover in which an electromagnetic absorber such as ferrite or the like or metal powder such as aluminum, stainless steel or the like is mixed into a resin material such as polypropylene or the like which has elasticity or bendability, and is formed. A development part 31 is so formed as to be developable in a direction indicated by arrows in Fig. 3.

As shown in Fig. 4A, a projection 32, for example, is formed on the side of one end face of the development part 31, and a recess 33 which is engaged with the projection 32 is formed on the side of the other end face. Specifically, engagement between the projection 32 and the recess 33 which are formed in the end faces of the development part 31 is detached whereby the electromagnetic shielding cover 30 is detachable with respect to the connector part 13. In this connection, the reference numeral 34 shows an electric-connector opening, the reference numeral 35 shows a suction base opening, and the reference numeral 36 is a gas-supply·water-supply base opening.

In this manner, the electromagnetic shielding cover is formed by a resin material having bending or flection ability, and is made to a construction which is developable in a longitudinal direction, whereby it is possible to provide an electromagnetic shielding cover which is easy in detachment with respect to the connector part. Thus, mounting ability or operability of the electromagnetic shielding cover to the connector part is considerably improved. The other functions and advantages are similar to those of the first enforcement form.

In connection with the above, a hinge part 31a is provided at a position which is opposed against the development part 31, as shown in Fig. 4B, whereby not only the development of the electromagnetic shielding cover 30 is more smoothed, but also the durability thereof can be improved.

A third embodiment of the invention will be described with reference to Figs. 5 and 6.

As shown in Fig. 5, an electromagnetic shielding cover 40 in the present embodiment is so arranged as to be able to be divided into a first shielding cover 41 and a second shielding cover 42, as shown in Figs. 6A and 6B. The electromagnetic first shielding cover 41 and the second shielding cover 42 are formed such that an electromagnetic absorber such as, for example, ferrite or the like, or metal powder such as aluminum, stainless steel or the like, is mixed into hard thermoplastic resin such as polysulfone, denaturated PPO, polyetherimide or the like, for example.

As shown in Figs. 6A, 6B and 6C, the first shielding cover 41 and the second shielding cover 42 are arranged such that a projection 43a, for example, provided on a divided face 43 of the first shielding cover 41, which is indicated by slash lines, and a recess 44a, for example, formed in a divided face 44 of the second shielding cover 42, which is indicated by slash lines, are engaged with each other to form the electromagnetic shielding cover 40 which is shown in Fig. 5.

In this manner, the electromagnetic shielding cover is divided by a plurality of members and is arranged whereby mounting and demounting with respect to the connector part are made further easy and universal. Thus, operability is improved. The other functions and advantages are similar to those of the aforementioned embodiment.

In connection with the above, even if the electromagnetic shielding cover is not so arranged as to combine two members with each other as described above, but to combine three or more members with each other to form the electromagnetic shielding cover, it is possible to obtain similar function and advantages.

A fourth embodiment of the invention will be described with reference to Fig. 7.

As shown in Fig. 7, an electromagnetic shielding cover 50 in the present embodiment is formed which is in the form of a resin sheet such as polyester, polyurethane or the like into which an electromagnetic absorber such as ferrite or the like or metal powder such as aluminum, stainless steel or the like is mixed, or in the form of a sheet in which a metal thin wire such as stainless steel or the like is wound.

The electromagnetic shielding cover 50 is formed therein with a connector opening 51 for the electric cord, an opening 52 on the side of the universal cord and an end-part opening 53. These openings 51, 52 and 53 are formed such that their respective inner diameter sizes are set larger than respective outer diameter sizes of the connector part 13 and the connector part 14 for the electric cord corresponding respectively to the openings 51, 52 and 53. Further, similarly to an inner diameter size of a core or drum 54 of the electromagnetic shielding cover 50, the inner diameter size of the core 54 of the electromagnetic shielding cover 50 is so formed by a size larger than the outer diameter size of the corresponding connector part 13.

In the opening vicinity of the connector opening 51 for the electric cord the opening 52 on the side of the universal cord and the end opening 53, an elastic or resilient member 55 is arranged which is provided with rubber, spring or the like which acts in a direction whose a diameter is contracted or reduced, with respect to an outer diameter size of the connector part 13 and the electric-cord connector part 14, which are arranged at the respective openings 51, 52 and 53.

With the arrangement, when the connector part 13, for example, is covered with the electromagnetic shielding cover 50, the inner diameter size of each part of the electromagnetic shielding cover 50 is formed larger than the outer diameter size of the connector part 13. Accordingly, mounting operation is executed smoothly. Moreover, after mounting of the electromagnetic shielding cover 50, the elastic member 55 which is arranged in the vicinity of the respective openings is contracted, whereby portions including the connector opening 51 for the electric cord, the opening 52 on the side of the universal cord and the end opening 53 are tightened.

In this manner, the elastic member is provided which is reduced in diameter less than outer diameters of respective members which are arranged on the respective openings in the vicinity of an opening in the electromagnetic shielding cover, and the connector part is tightened by the elastic member so as to be fixed thereby, whereby shift or deviation during operation is eliminated. Thus, it is possible to hold or retain shielding effects during the use. Furthermore, the whole or entire electromagnetic shielding cover is formed by shape memory resin into which the electromagnetic absorber such as ferrite or the like, or metal powder such as aluminum, stainless steel or the like is mixed. Thus, after the electromagnetic shielding cover has been mounted on the connector part, heat or the like is applied to the electromagnetic shielding cover so as to deform the same, whereby it is possible to fix the electromagnetic shielding cover to the connector part more reliably. The other function and advantages are similar to those of the aforementioned enforcement form.

As shown in Fig. 1, the electric-cord connector 16 to which the electric cord 15 detachably connected to the video processor 4 is connected is provided on the side of the connector part 13. Since the electric-cord connector 16 is provided with the group of contact pins, the electric-cord connector 16 is efficiently shielded whereby the shielding ability of the connector part 13 is considerably improved. Here, radiative-noise reduction countermeasures of the electric-cord connector 16 of the connector part 13 will be described.

A fifth embodiment of the invention will be described with reference to Fig. 8.

As shown in Fig. 8, the electric-cord connector part 61 which is provided on the side of a connector part 60 in the present embodiment comprises an electric connector body 62, a casing 63 of the connector part 60, which is formed by synthetic resin such as polysulfone, denaturated PPO, polyetherimide or the like, and a shielding case 64 arranged between the electric connector body 62 and the casing 63 and formed by an electromagnetic absorber, a metal conductor, a metal mesh or net wire or the like as a radiative-noise reduction member which wraps the electric connector body. In this connection, the reference numeral 65 is a substrate for fixing the group of contact pins 23.

In this manner, the shielding case which wraps the outer peripheral part of the electric connector body is interposed with respect to the casing, whereby it is possible to efficiently absorb and cut the noises from the electric connector body which most radiates the noises. Further, since the shielding case is interposed between the casing and the electric connector body, it is possible to efficiently absorb the radiative noises, irrespectively of a material of the casing.

A sixth embodiment of the invention will be described with reference to Fig. 9.

As shown in the Fig. 9, in the present embodiment, when a space part 66 which is partitioned by the substrate 65 which fixes the group of contact pins 23 of the electric connector body 62 is provided with a signal cable 25 or the contact pin 21, the space part 66 is filled with powder 67 consisting of an electromagnetic absorber such as ferrite or the like. Thus, it is possible to absorb and cut radiative noises which are generated from the contact pins 23 and the signal cable 25.

In connection with the above, by the fact that the substrate 65 is formed by a resin material into which the electromagnetic absorber such as ferrite or the like is mixed, it is possible to further efficiently and effectively absorb and cut the radiative noises which are generated from the contact pin 23 and the signal cable 25. Moreover, even if the electric connector 16 which is provided on the electric cord which is connected to the electric connector body 62 of the electric-cord connector part 61 is filled with the powder 67 consisting of an electromagnetic absorber such as ferrite or the like, it is possible to obtain similar function and advantages. Furthermore, as shown in Fig. 10, the powder electromagnetic absorber 62 such as ferrite or the like as indicated by a two-dot-and-chain line in the figure is coated on the electric connector body 62 and an inner face of the electric connector body 62 in the present embodiment, and the group of contact pins 23 is fixed to the substrate 65 by adhesion. At this time, a powder electromagnetic absorber such as ferrite or the like is mixed into insulation adhesives 68 which are poured in order to fix the group of contact pins 23 and the substrate 65 to each other, whereby it is possible to obtain function and advantages similar to those of the sixth embodiment.

The following electronic endoscope apparatus will be described. That is, as described above, since there are many connector parts which are made of a resin material, the light source device is arranged as follows, to cover the connector part, in place of the fact that the connector part is covered with the electromagnetic shielding cover, whereby it is possible to easily conduct the EMC countermeasures, irrespectively of the material and the construction which form the connector part.

A seventh embodiment of the invention will be described with reference to Fig. 11.

As shown in Fig. 11, in the present embodiment, an outer periphery of the connector part 13 is surrounded by a casing for a light source device 3a that is a surrounding member made of a metal in place of the fact that the connector part 13 is covered with the electromagnetic shielding cover. Specifically, a detachable part 71 with respect to the connector part 13, which is provided on the light source device 3a is located at a position which is gotten into the internal side of the light source device 3a more than a front panel 72. An amount of displacement by which the light source device 3a is gotten into the internal side from the front panel 72 at this time is set such that, when the connector part 13 is mounted to the detachable part 71, the whole of the connector part 13 is contented in the internal side of a casing for the light source device 3a. The casing of the light source device 3a is formed by a metal material having conductivity such as, for example, aluminum, iron or the like, and is grounded.

In this manner, the detachable part with respect to the connector part is so arranged as to be provided at the position gotten into the internal side of the light source device more than the front panel of the light source device, whereby, when the connector part is connected to the light source device, the connector part becomes a state which is housed or received into the casing of the light source device. Thus, it is possible to electromagnetically shield the connector part by the casing made of a metal.

Further, as shown in Fig. 12, a tubular shielding wall 74 which is formed by a metal material having conductivity, such as, for example, aluminum, iron or the like may be arranged on a periphery of a detachable part 73 of the connector part 13 which is provided on a light source device 3b. At this time, an inner diameter size and a height of the shielding wall 74 is set to a size or dimension in which, when the connector part 13 is mounted to the detachable part 73, the connector part 13 is received within the shielding wall 74. Furthermore, the shielding wall 74 is grounded. An opening 74a, on the side of hand, in the shielding wall 74 is made to an opening size which is reduced in diameter to such a degree that the connector part 13 can pass therethrough.

In this manner, the shielding wall made of the metal, which receives the connector part mounted to the detachable part of the light source device, is arranged whereby the circumstances of the connector part are electromagnetically shielded by the shielding wall. Thus, it is possible to obtain advantages similar to those of the seventh embodiment. Further, since the shielding wall projects more than the front panel of the light source device, cleaning or scavenging ability of the surface of the light source device, and maintenance ability of the light source device are improved.

In connection with the above, as shown in Fig. 13, a threaded engagement part 75 is formed on the shielding wall 74 and the light source device 3a so that they are detachable from each other, whereby, when the connector part 13 and the light source device 3a are detached, the shielding wall 74 is detachable or removable from the light source device 3a. Thus, it is possible to remarkably improve detachability between the connector part 13 and the light source device 3a.

An electromagnetic interference countermeasure member which covers the universal code 12 having the connector part 13 at an end thereof will subsequently be described.

An eighth embodiment of the invention will be described with reference to Figs. 14 to 16.

As shown in Fig. 14, the electronic endoscope 2 comprises the elongated insertion part 10 having elasticity, the operation part 11 formed at a proximal end of the insertion part 10, the universal cord 12 which extends from the operation part 11, and the connector part 13 which is provided at an end of the universal code 12.

The connector part 13 is provided with an electric connector part 14 that is an electric connection part provided with the group of contact pins 23, which is connected to the video processor 4 which is an external equipment, through the electric cord 15, a light-guide incidence end 112, a gas supply base 113, and the like.

As shown in Fig. 15, the forward-end forming part 7 is provided, at a forward-end face thereof, with a nozzle 114, an observation window 115 arranged in opposed relation to the nozzle 114, two illumination windows 116 and a forceps outlet 117.

As shown in Fig. 14, the nozzle 114 is connected to a gas-supply·water-supply tube 118 within the curvature part 8 continuous to the forward-end forming part 7. The gas-supply·water-supply tube 118 branches into a water supply tube 119 and a gas supply tube 121 within the flexible pipe part 9. Under this state, the gas-supply·water-supply tube 118 is inserted and passes through the operation part 11 and the universal cord part 12. At the connector part 13, the gas supply tube 121 is connected to the gas supply base 113, while the water supply tube 119 is connected to an unshown water supply base.

Illumination lenses 124 are mounted respectively to the two illumination windows 116 which are formed in the forward-end forming part 7. Light guide fiber bundles 125 have their respective forward ends which are fixed respectively to the interiors of the illumination lenses 124.

As shown in Fig. 16, the light guide fiber bundles 125 have respective outer peripheral faces thereof which are covered with and protected by light-guide protection tubes 126, respectively. The light guide fiber bundles 125 are inserted and pass through the curvature part 8, the flexible pipe part 9, the operation part 11 and the universal cord 12 under a state covered with these light guide protection tubes 126. The light guide fiber bundles 125 have rearward ends thereof which reach the light-guide incidence end 112 which is provided on the connector part 13. The light-guide incidence end 112 is connected to the light source device 3, whereby the light guide fiber bundles 125 transmit the illumination light, output the same from the illumination windows 116 and illuminate an object such as an affected or diseased part or the like.

The illuminated object focuses into an image at an image formation or imaging position by an objective optical system 127 which is mounted to the observation window 115 which is formed between the two illumination windows 116 in the forward-end forming part 7. At the imaging position, a solid-state image pickup element 128 such as a CCD or the like is arranged to photoelectrically convert the imaged optical image.

As shown in Fig. 14, the objective optical system 127 is arranged such that the observation window 115 is blocked or closed by a first lens, and the solid-state image pickup element 128 is positioned and fixed to the final lens which forms the objective optical system 127. The solid-state image pickup element 128 is so arranged as to be packaged. A lead is provided in projection on a rear face of an image pickup face. A first circuit substrate 129 and a second circuit substrate 130 are connected to the lead. The first circuit substrate 129 packages a chip condenser or capacitor and two ICs on a ceramic substrate.

One of the ICs is for generating a drive signal which drives the solid-state image pickup element 128, while the other IC is for generating a video signal. The first circuit substrate 129 and the second circuit substrate 130 which extend to the rearward side have their respective rearward ends which are connected to an image pickup cable 132 which is fixed by a cable fixing block 131. Further, a GND of each of the circuit substrates 129 and 130 is conducted to the cable fixing block 131 made of a metal.

A power source for the solid-state image pickup element 128, and the image pickup cable 132 which is formed by a plurality of coaxial lines which execute giving and receiving of the signal and/or a single wire comprise a first metal conductor 133, three coaxial lines including a first insulation resin layer 134, a second metal conductive layer 135 and a second insulation resin layer 136, a third metal conductive layer 137 which surrounds the same and which lumps together, and a third insulation resin layer 138, for example.

Here, the first insulation resin layer 134, the second insulation resin layer 136 and the third insulation resin layer 138 are arranged such that ferrite particles are mixed into resin such as Teflon, polyvinyl chloride or the like. The first metal conductor 133 of the image pickup cable 132 is soldered to the first circuit substrate 129 and the second circuit substrate 130. The second metal conductive layer 135 is soldered to the cable fixing block 131.

In connection with the above, the third metal conductive layer 137 is not connected within the forward-end forming part 7. A rearward end part of the third metal conductive layer 137 and an end face of the third insulation resin layer 138 are so adjusted as to stay or to be contented within a first curvature frame or piece 142 which forms the curvature part 8. The third metal conductive layer 137 is covered with a heat-contraction tube 141 from a position above the solid-state image pickup element 128, the first circuit substrate 129 or the like. The interior of the third metal conductive layer 137 is filled with epoxy resin having insulation. Thus, the third metal conductive layer 137 is fixed.

The curvature part 8 comprises the first curvature piece 142, connection curvature pieces 143, a final curvature piece 144 and a tubular curvature-part covering rubber 145 which covers them. The first curvature piece 142 is fixed to the forward-end forming part 7, while the final curvature piece 144 is fixed to the flexible pipe part 9. The second insulation resin layer 136 and the connection curvature pieces 143, the connection curvature pieces 143 and the final curvature piece 144, and the connection curvature pieces 143 and the connection curvature pieces 143 are connected to each other for angular movement.

A curvature mechanism is formed in which an unshown angle knob which is provided on the operation part 11 is operated whereby angle wires which are connected to the angle knob are pushed and pulled (or moved forward and rearward) so as to be capable of curving the curvature part 8 in optional directions including an upper direction, a lower direction, a left-hand direction and a right-hand direction. As shown in Fig. 16, angle wires 140 are arranged within the insertion part 10 respectively along four directions, and have forward ends thereof which are fixed to the first curvature piece 142.

The image pickup cable 132 is arranged such that, within the flexible pipe part 9, the operation part 11, the universal cord 12 and the connector part 13, shielding tapes 146 in which a conductive paint 146a is applied to one face of a strip- or strap-like member which is wide in width, which is formed by an insulation resin material is wound further from the upper part of the third insulation resin layer 138. At this time, a conductive face is made to one on the side of the third insulation resin layer 138, and the opposite face which becomes the outside is insulated. A jumper wire 147 is so drawn out as to be conducted to the conductive paint 146a, from the end face within the connector part 13 so that the shielding tapes 146 are connected to a metal blade 148 on an inner face of the universal cord part 12.

The metal blade 148 is provided on portions of the flexible pipe part 9, the operation part 11, the universal cord 12 and the connector part 13, and they are all conducted to each other. The third insulation resin layer 138 is arranged such that, within the connector part 13, the image pickup cable 132 is formed or molded in strip similarly to the inside of the curvature part 8, and the first metal conductor 133, the second metal conductive layer 135 and the third metal conductive layer 137 are connected to the contact pins of the group of contact pins 23.

Moreover, the operation part 11 is provided with a plurality of operation switches 149 which execute indication such as freeze or the like. Each of the operation switches 149 is connected to an associated switch cable 150. These switch cables 150 are inserted and pass through the interior of the universal cord 12, under a twisted or twined state, for example. Within the connector part 13, each of the switch cables 150 is fixed in soldering to the associated one of the contact pins similarly to the image pickup cable 132.

The contact pins of the group of contact pins 23 are connected to the video processor 4 through the electric cord 15. In the present embodiment, the universal cord 12 is covered with a universal cord cover 152.

The universal cord cover 152 is made of extendible and retractable bellows-like insulation resin. An inner face thereof is provided with a metal evaporation layer 153 which serves as an electromagnetic interference countermeasure member.

A fixing part 154 on the side of the connector is provided on the side of the connector part 13 of the universal cord cover 152, and a fixing part 155 on the side of the operation part is provided on the side of the operation part 11. The fixing part 154 on the side of the connector and the fixing part 155 on the side of the operation part are made of rubber or spring which is reduced in diameter similarly to the elastic member 55 which is arranged in the vicinity of the opening illustrated in Fig. 7. The fixing part 154 and the fixing part 155 are so arranged as to firmly be fixed to the connector part 13 and the operation part 11, respectively.

Furthermore, an earth line 156 comes out from the metal evaporation layer 153 of the fixing part 154 on the side of the connector. Thus, the arrangement is such that the earth line 156 can be conducted to the GND or an earth (ground) which is provided separately.

Furthermore, as shown in Fig. 16, the image pickup cable 132 is arranged substantially or generally at a center within the flexible pipe part 9. So as to surround the same, a forceps insertion and passage tube 123, the light-guide protection tubes 126, the water supply tube 119 and the gas supply tube 121, which are formed by the electromagnetic absorber, are arranged.

In this manner, the universal cord cover having the shielding member is provided on the universal cord, whereby it is possible to reduce radiation of the radiative noises from the image pickup cable and the switch cables, and mixture of the noises to the image pickup cable and the switch cables. Furthermore, since the universal cord cover is formed into the form of bellows, there is less probability that the elasticity of the universal cord is hurt or damaged. Further, since the universal cord cover is detachable with respect to the universal cord, sufficient shielding effects can be expected also with respect to an arrangement in which the shielding countermeasures are not applied to the electronic endoscope body. Since the earth line is provided on the universal cord cover per se, it can cope also with or deal also with any endoscopes and systems.

Moreover, since it is detachable, if the universal cord cover is mounted to the universal cod when it is used in combination with a plurality of equipments, particularly, equipments easy to receive influence of the noises or equipments easy to generate the noises, it is possible to simply or easily conduct or perform effective EMC countermeasures. At the time other than the same, if the electronic endoscope is used without the fact that the universal cord cover is mounted, it is possible to use the same as the normal or ordinary universal cord. That is, it is possible to be used as an electronic endoscope having the universal cord which is superior in operability, having sufficient elasticity.

Moreover, if the universal cord cover is used as disposable or throwaway one, it is preferable also from health or sanitation point of view.

Furthermore, the arrangement is such that a portion of the image pickup cable from the insertion part to the connector part is covered with the shielding tape which serves or functions as the electromagnetic interference or disturbance countermeasure member, and this is held or maintained to the electric potential the same as that of the metal blade which serves as the armor metal for the electronic endoscope. Accordingly, the shielding advantages further increase. On one hand, since the shielding tape is not provided on the curvature part, a fill rate or ratio within the curvature part does not rise, and no interference is made to the curvature operation. In this connection, even if the fill ratio rises more or less within the flexible pipe part and the universal cord, since tight turning is not required as the curvature part, there is no problem. Since conduction is made within the connector part with respect to the armor metal, operability is improved.

Furthermore, the arrangement or construction is such that the image pickup cable is arranged substantially at the center within the flexible pipe part, and the visceral objects including the electromagnetic absorber are arranged around the image pickup cable. Accordingly, the circumference or periphery of the image pickup cable becomes a state surrounded by the shielding member. Thus, the shielding advantages increase. In this connection, since the electromagnetic absorber such as ferrite or the like is included or contained within each of the insulation resins of the image pickup cable, the shielding advantage is increased.

A ninth embodiment of the invention will be described with reference to Figs. 17 and 18.

As shown in Fig. 17, an enlargement objective optical system 162, for example, is provided on the observation window 115 of the forward-end forming part 7. The enlargement objective optical system 162 is an objective optical system which has a movable lens so that enlarged observation is possible. The enlargement objective optical system 162 is image-formed to an unshown solid-state image pickup element within an image pickup part 163. The image pickup part 163 is arranged as a unit which includes the solid-state image pickup element 128, the first circuit substrate 129 and the like.

The movable lens which forms the enlargement objective optical system 162 is movable by the fact that a focus wire 165 within a focus-wire protection pipe 164 is pushed and pulled. The focus wire 165 is operated by a focus switch 166 which is provided on the operation part 11. The focus switch 166 is connected to the contact pins within the connector part 13 through the switch cables 150 which are inserted and pass through the universal cord part 12.

The image pickup part 163 is fixed to the forward-end forming part 7. However, the circumstances thereof are filled with a solid lubricant 167 having mixed thereto the ferrite particles. Further, the two image pickup cables 132 and 132 are connected to the image pickup part 163.

As shown in Fig. 18, the image pickup cable 132 comprises four coaxial cables, the third metal conductive layer 137 which covers the outside thereof, and the third insulation resin layer 138. However, the image pickup cable may comprise, not only the coaxial cable, but also combination of single wires each of which comprises a conductor and an insulation coating or covering.

The image pickup cables 132 are arranged such that, within the flexible pipe part 9, the operation part 11 and the universal cord 12, these two image pickup cables 132 are put together, and the put-together cables are covered with an electromagnetic disturbance or countermeasure cover 168 that is a magnetic interference countermeasure member, from the outside thereof. The image pickup cables 132 are connected to the contact pins which are provided on the electric contact part 11, within the connector part 13.

The electromagnetic interference countermeasure cover 168 is arranged such that metal thin wires are knitted, and a cross angle upon the fact that the metal thin wire is knitted is 90°. The electromagnetic interference countermeasure cover 168 is arranged such that a forward-end jumper wire 169 is drawn out from an end of the electromagnetic interference countermeasure cover 168 on the side of the curvature part 8, that is, a forward end of the electromagnetic interference countermeasure cover 168 and is connected to the end, on the side of the flexible pipe part or elastic tube 9, of a curvature-part metal blade 170 which covers the curvature pieces 142, 143 and 144 of the curvature part 8. Further, a rearward-end jumper line 171 is drawn out from the end of the electromagnetic interference countermeasure cover 168, and is connected to an inner face of a universal-cord-cover conduction part 172 made of a metal.

The curvature-part metal blade 170, a flexible-pipe-part metal blade 173, an operation-part metal part 174, a universal-cord metal blade 175 and a connector-part metal part 176 are connected at respective end parts thereof to each other, and are conducted to each other.

The curvature-part metal blade 170 has an outer peripheral face thereof which is covered with the curvature rubber 145. The flexible-pipe-part metal blade 173 is covered with an elastic pipe resin tube 177 which becomes an armor member for the flexible pipe part 9. The operation-part metal part 174 is covered with an operation-part armor member 178 which is formed by resin or the like. The universal-cord metal blade 175 is covered with an electric universal-cord resin tube 179 which serves as an armor member for the universal cord 12. The universal-cord resin tube 179 has a rearward end thereof which is fixed to a metallic armor member 180 of the connector part 13.

A universal-cord-cover conductive part 181 which draws out the operation-part metal part 174 on the inner face of the operation part 11, to the outer face of the operation part 11 is provided adjacent to the operation-part metal part 174 on the side of the universal cord 12. Similarly, the universal-cord-cover conduction part 172 which draws the connector-part metal part 176 on the inner face of the connector part 13, out to the outer face of the connector part 13, and which is conducted to a conductive member of a universal cord cover 182 is provided adjacent to the electric contact part 11 of the connector-part metal part 176.

The universal cord cover 182 which covers the universal cord 12 is made of elastic insulation resin. A universal-cord-cover metal blade 183 is laminated upon the inner face of the universal cord cover 182. The universal cord cover 182 has both end parts thereof at which a fixing part 84 on the side of the operation part and a fixing part 85 on the side of the connector are provided respectively, and which are so arranged as to be detachable with respect to the operation part 11 and the connector part 13.

Upon mounting of the universal cord cover 182, the universal-cord-cover metal blade 83 is respectively in contact with the universal-cord-cover conduction part 172 and the universal-cord-cover conductive part 181 and is conducted thereto. In connection with the above, as shown in Fig. 18, a forward water-supply tube 186 is provided within the insertion part 10.

With the above-described arrangement, it is possible to make the curvature-part metal blade 170, the flexible-pipe-part metal blade 173, the operation-part metal part 174, the universal-cord metal blade 175 and the connector-part metal part 176, the electromagnetic interference countermeasure cover 168, and the universal-cord-cover metal blade 183 of the universal cord cover 182, to the same potential.

Further, as shown in Fig. 18, the image pickup cables 132 are arranged substantially at a center within the flexible pipe part 9, and the forceps insertion and passage tube 123, the light-guide protection tubes 126, the water supply tube 119, the gas supply tube 121, the forward water-supply tube 186, and the focusing-wire protection pipe 164, which are made of the electromagnetic absorber, are arranged so as to surround the same. The forward water-supply tube 186 is a pipe line for supplying water forwardly, which is many in being used in an endoscope for a lower-part digestive organ.

In this manner, since a portion which is conducted to the armor metal is provided at a portion at which the universal cord cover is mounted, it is possible to be made simply to the potential the same as those of the universal cord cover and the armor metal. Thus, the shielding advantages are improved. Moreover, although it becomes a state floated potentially as it is, if the jumper line is drawn out from the end part of the universal cord cover, it is possible to simply be made to GND.

Furthermore, also even at the time when the universal cord cover is not mounted, it is possible to simply make the armor metal to the GND by a metal portion of the mounting portion.

Moreover, since the image pickup cable is covered with the electromagnetic interference countermeasure cover with two collected together, handling of the image pickup cable is ease or comfort. Further, since the electromagnetic interference countermeasure cover is conducted at two locations before and behind the same, a conductive state is further stabilized, and, even in a case where one of them is unfastened, it is possible to maintain the shielding function. In this connection, by the fact that, if the visceral objects within the insertion part increase, it is only to use the tube into which the electromagnetic absorber is mixed, it is possible to obtain the shielding advantages without being large-sized in diameter.

In connection with the above, the invention is made to, for example, a detachable arrangement in which the universal cord cover 152 in the eighth embodiment can be mounted to and can be demounted from the universal cord 12 from the side of the connector part 13 or the side of the insertion part 10. However, the detachable arrangement includes, for example, also an arrangement in which the universal cord cover 152 is fixed in the vicinity of the connector part 13 such that the bellows is folded up, and, in a case where the universal cord 12 is required to be covered, the bellows extends, and the extended end part, that is, the fixing part 155 on the side of the operation part is fixed in the vicinity of the operation part 11.

Furthermore, in a case where the universal cord cover 152 is fixed in such a manner that the bellows is folded up in the vicinity of the connector part 13, it is made possible to use it under a state apt to be used, similarly to the usual or normal electronic endoscope.

The arrangement may be such that means for simply releasably folding up the universal cord cover 152 to, for example, the connector part 13 or the like to fix or receive the same is provided so that the universal cord cover 152 is held under a fixed state in accordance with the use environment or the like, the fixing is released, and the bellows extends as described above, to fix the fixing part 155 on the side of the operation part, in the vicinity of the operation part 11.

In connection with the above, the present invention is not limited to the electronic endoscope in which the image pickup means is built in the forward end part of the insertion part and which has the universal cord through which is inserted and passes the image pickup cable connected to the image pickup means. The invention is applicable also to a case of a TV-camera equipped endoscope in which a TV camera which has built therein image pickup means such as a solid-state image pickup element or the like is equipped in an ocular part of an optical endoscope which has an image guide.

Specifically, the invention is applicable also to an arrangement in which a cord which is generally called "camera cord" having one end thereof extending from a TV camera and the other end at which a connector connected to a video processor or a camera control unit is provided and through which an image pickup cable connected to image pickup means within the TV camera is inserted and passes can detachably be covered by a cord cover which has electromagnetic interference countermeasure function.

A tenth embodiment of the invention will be described with reference to Figs. 19 to 24.

As shown in Fig. 19, the electronic endoscope 2 according to the present embodiment is arranged such that a universal cord 212 and a signal cable 214 extend therefrom, the universal cord 212 having built therein a gas supply tube, a water supply tube, a light guide for transmitting an illumination light from an end of the operation part 11, and various kinds of signal lines being inserted and passing through the signal cable 214. A connector 213 for the light source which is detachable to the light source device 3 is provided at an end of the universal cord 212. A metal electric connector 215 having a structure or an armor portion which can detachably be mounted to the video processor 4 is provided on an end of the signal cable 214.

In connection with the above, the side of the operation part 11 that is the proximal end of the signal cable 214 is formed as shown in Fig. 20. That is, as shown in Fig. 20, a base 218 made of a metal material is integrally fixed to the end of the signal cable 214 by adhesion or the like. An O-ring 219 is provided around an outer periphery of the base 218. Furthermore, an abutment 221 which is abutted against a step 220 which is formed on the base 218 and a connection member 223 having, on an inner peripheral face thereof, a threaded part 222 are inserted outwardly on the outer periphery of the base 218. Moreover, a plurality of electric contacts 224 are fixed to the inner peripheral side of the base 218, through a fixing plate 225. A core wire 226 within the signal cable 214 is fixed to the associated one end of each of the electric contacts 224 by solder or adhesives having electric conductivity. In connection with this, in the present figure, the core wire 226 is illustrated as being a single line, but may be a coaxial line.

On one hand, the operation part 11 is provided with a signal-cable receipt base 227. The signal-cable receipt base 227 has an outer periphery thereof at which a threaded part 228 is provided which is threadedly engaged with the threaded part 222. The signal-cable receipt base 227 is provided on the inner periphery thereof with contact pins 231 which are engageable respectively with the electric contacts 224 through a fixing plate 229 and a fixing plate 230.

As shown in Fig. 21, in the signal cable 214, each of a first shielding layer 241 and a second shielding layer 242 is provided at an outer periphery of a bundle which is formed in such a manner that the core wire 226 is twisted or twined. These first shielding layer 241 and second shielding layer 242 are arranged such that metal thin conductors or lead wires 243 such as, for example, a copper wire, a copper alloy wire and stainless steel are spirally wound in the same direction. In this manner, the first shielding layer 241 and the second shielding layer 242 are so arranged as to be wound in the same direction whereby the conductors 243 which are double piled up to each other are piled to each other without a gap as shown in an enlarged view in Fig. 21. Thus, the arrangement is such that it is secured that the noises which leak from the gap between the first shielding layer 241 and the second shielding layer 242 are cut. At this time, of the plurality of core wires 226, a signal line that is high in drive frequency, that is, a signal line which is easy to send or put out the noises, for example, a horizontal-drive-pulse line or the like is twisted or twined and bundled such that the pulse line becomes a center of the bundle, whereby the signal line is shielded by another core wire 226, so that the arrangement is such that it is difficult for noises to go out.

Furthermore, the signal cable 214 is covered with an insulation jacket 244 which is made of 4-6 fluoride resin, or 4-fluoride ethylene perfluoro alkoxy ethylene copolymer resin.

Moreover, one ends of the first shielding layer 241 and the second shielding layer 242 are connected to a video ground of the video processor 4 and the other ends thereof may be connected to a video ground of the CCD 10 at the forward end of the electronic endoscope 2, through the electric contacts 224 and the contact pins 231.

Furthermore, in order to reduce the radiative noises from the peripheral devices such as the light source device 3, the video processor 4 and the like, as shown in Fig. 19, the casing of the light source device 3, the casing of the video processor 4 and the cart 6 are conducted to each other through conductive materials 245 which are provided on the casing of the light source device 3 and the casing of the video processor 4 and are grounded to the earth.

Furthermore, the arrangement is as follows. That is, in order to change or alter efficiency which reduces the radiative noises in accordance with the external environments at a location where the endoscope apparatus 1 is used, at least two kinds of electromagnetic absorber members which are different only in wall thickness, including a first electromagnetic absorber member 262 made of an electromagnetic absorber such as ferrite, for example, provided with a cut-out 261 whose inner diameter as shown in Fig. 22 is substantially equal in diameter to an outer diameter ⌀A of the signal cable 214 and whose wall thickness is x1, a second electromagnetic absorber member 263 made of an electromagnetic absorber which has the cut-out 261, which is different only in the wall thickness from the first electromagnetic absorber member 262, which is substantially equal in diameter as the outer diameter ⌀A of the signal cable 214 shown, for example, in Fig. 23, whose wall thickness is x2 are prepared, and the like, whereby the electromagnetic absorber member whose wall thickness is adequate is selected by the external environment, and, as shown in Fig. 24, the electromagnetic absorption member is arranged at the signal cable 214 through the cut-out 261 and is used.

In this manner, the signal cable is provided exclusive for signal transmission, whereby it is possible sufficiently to conduct the EMC countermeasures to the signal cable. Further, since it is arranged detachably to the signal cable through the base which is separate from the electronic endoscope and which is made of a metal, specific or special EMC countermeasures are unnecessary to be conducted to the electronic endoscope and the universal code. Thus, it is made possible to realize the electronic endoscope apparatus in which assembling is made easy and which is low in cost.

Furthermore, the electromagnetic absorber member is suitably arranged at the signal cable in accordance with the external environments whereby it is possible to reduce radiation of the unnecessary radiative noises from the signal cable and mixture of the unnecessary radiative noises to the signal cable.

Further, the signal cable is arranged such that the base is inserted into the signal-cable receipt base, and is threadedly engaged therewith, whereby the electric contact and the contact pins are connected to each other and are electrically conducted to each other. Thus, it is possible to water-tightly and detachably fix the signal cable to the operation part by the O-ring.

An eleventh embodiment of the invention will be described with reference to Figs. 25 and 26.

As shown in Fig. 25, a signal cable 214a in the present embodiment is set such that a diameter size or dimension of the lead wire 73 of the double-layer first shielding layer 71 having the metal conductivity, which is wound in the same direction and a diameter size of the lead wire 73 of the second shielding layer 72 are set different from each other. Moreover, the insulation layer 75 made of synthetic resin such as 4-fluoride ethylene resin or the like is provided between the first shielding layer 71 and the second shielding layer 72. The other arrangement is similar to that of the tenth embodiment,

Generally, there is the relationship between the diameter size of the conductor or the lead wire and the frequency of the noises which can be excluded that it is possible to exclude a frequency from a low frequency to a high frequency in accordance with the fact that the diameter size of the element wire increases. Moreover, as shown in Fig. 26, when the diameter size of the lead wire is ⌀X and ⌀XY, for example, frequencies f1 and f2 which can efficiently be excluded are different from each other.

In the present embodiment, the insulation layer is interposed between the first shielding layer and the second shielding layer which cooperate with each other to form the signal cable, and the first shielding layer and the insulation layer are made, respectively, to layers independent of each other. Thus, a difference in diameter size between the lead wire of the first shielding layer and the lead wire of the second shielding layer appears or comes out clearly. Accordingly, in addition to the advantages of the tenth embodiment, the characteristics shown in Fig. 26 are utilized, and a broader or wider frequency region is excluded by one of the two lead wires which have diameters thereof different from each other, and a frequency of noises due to an electrocautery, for example, 300 ∼ 600 kHz, is the most well excluded by the other lead wire. Thus, it is possible to excuse the noise countermeasure more efficiently.

Lastly, an electromagnetic interference countermeasure member which covers an external device to which the connector part is connected, according to a twelfth embodiment of the invention will be described with reference to Fig. 27.

As shown in Fig. 27, in the present enforcement form, as the EMC countermeasures for the peripheral equipments of the electronic endoscope apparatus 1 in Fig. 1 or 19, which is provided with the electronic endoscope, the cart 6 and the periphery of the monitor 5 are covered with a first shielding curtain 251 which is so formed, for example, as to be wound by stainless metal element wires, on one hand, front faces of the light source device 3 and the video processor 4 are covered with a second shielding curtain 252 which is wound similarly to a first shielding curtain 351. An upper face of a keyboard 254 which inputs the indication with respect to the video processor 4 is covered with a keyboard cover which is made of an electromagnetic absorber such as, for example, ferrite, or is covered with a third shielding curtain 253 which is wound similarly to the first shielding curtain 251.

In this manner, by the fact that the external device which forms the electronic endoscope apparatus is covered with the shielding curtain, it is possible to reduce radiation of the unnecessary radiative noises and mixture of the unnecessary radiative noises. Thus, by the fact that the shielding curtain is held always available as a member which forms the electronic endoscope apparatus, when it is used in combination with an equipment which is apt to receive affection of the noises, or an equipment which is apt to generate the noises, if the shielding curtain is covered on the external device which is formed as a system as occasion demands, it is possible to easily conduct the effective EMC countermeasures. At the time other than the same, the external device is used without the fact that the shielding curtain is mounted thereon.

In the present invention, it is apparent that working modes or embodiments different in a wide range can be formed on the basis of this invention without departing from the spirit and scope of the invention. The present invention is not restricted by any specific embodiment being limited by the appended claims.

## Claims

1. An electronic endoscope apparatus comprising:
an electronic endoscope having arranged at an insertion part thereof a solid-state image pickup element as image pickup means; and
external devices such as a light source device for supplying an illumination light to the electronic endoscope, a video processor for processing an electric signal which is transmitted from the solid-state image pickup element of said electronic endoscope, to a video signal, and the like,
wherein an electric connection part for electrically connecting said electronic endoscope and said video processor to each other is provided on a connector part which is provided at an end of a universal cord which connects said electronic endoscope and the light source device to each other, and
wherein said electronic endoscope apparatus is provided with an electromagnetic interference countermeasure member which covers at least one of said connector part, said universal cord and said external devices.

2. An electronic endoscope apparatus according to claim 1, wherein
an electromagnetic shielding cover is provided as the electromagnetic interference countermeasure member which covers an armor of said connector part.

3. An electronic endoscope apparatus according to claim 2, wherein said electromagnetic shielding cover is detachable with respect to said connector part.

4. An electronic endoscope apparatus according to claim 3, wherein said electromagnetic shielding cover is formed by at least two electromagnetic shielding covers, and wherein said two electromagnetic shielding covers are combined with each other to cover said connector part.

5. An electronic endoscope apparatus according to claim 3, wherein said electromagnetic shielding cover has a development part over a whole length thereof in a longitudinal direction, and is developable at the development part.

6. An electronic endoscope apparatus according to claim 3, wherein said electromagnetic shielding cover is provided with a hinge part.

7. An electronic endoscope apparatus according to claim 3, wherein, in the vicinity of an opening in said electromagnetic shielding cover, an elastic member for reducing in diameter the opening is provided.

8. An electronic endoscope apparatus according to claim 2, wherein said electromagnetic shielding cover is formed by a resin material which contains an electromagnetic absorber.

9. An electronic endoscope apparatus according to claim 2, wherein said electromagnetic shielding cover is formed by the fact that a metal thin wire is wound.

10. An electronic endoscope apparatus according to claim 2, wherein said electromagnetic shielding cover is formed by an electromagnetic absorber.

11. An electronic endoscope apparatus according to claim 1, wherein a radiative-noise reduction member is further provided at the electric connection part which electrically connects said electronic endoscope and the video processor to each other.

12. An electronic endoscope apparatus according to claim 11, wherein said radiative-noise reduction member is one of a shielding case which is formed by one of an electromagnetic absorber, a metal conductor and a metal net wire, and which covers an electric connector body which forms the electric connection part, and a shielding case into which said electromagnetic absorber is mixed.

13. An electronic endoscope apparatus according to claim 11, wherein said radiative-noise reduction member is powder consisting of an electromagnetic absorber which is filled in a space part in an electric connector body.

14. An electronic endoscope apparatus according to claim 11, wherein said radiative-noise reduction member is a coating part which has an electromagnetic absorber which is applied to an inner face of the electric connector part.

15. An electronic endoscope apparatus according to claim 11, wherein said radiative-noise reduction member is an electromagnetic absorber which is mixed into an adhesive which fixes a contact pin which is provided within the electric connector part.

16. An electronic endoscope apparatus according to any one of claim 8, claim 10, claim 12, claim 13, claim 14 and claim 15, wherein said electromagnetic absorber is ferrite.

17. An electronic endoscope apparatus according to claim 1, wherein said electromagnetic interference countermeasure member is a surrounding member made of a metal, which is so arranged as to surround an outer periphery of said connector part, and wherein said surrounding member made of the metal is grounded.

18. An electronic endoscope apparatus according to claim 17, wherein said surrounding member made of the metal is a light source device, wherein a detachable part with respect to said connector part is provided within said light source device, and wherein the outer periphery of said connector part is surrounded by the light source device.

19. An electronic endoscope apparatus according to claim 17, wherein said surrounding member made of the metal is a tube which is provided in projection from the light source device.

20. An electronic endoscope apparatus according to claim 19, wherein said tube is detachable with respect to said light source device.

21. An electronic endoscope apparatus according to claim 1, wherein a universal cord cover which is in the form of a tube and which has conductivity is detachably provided as the electromagnetic interference countermeasure member which covers the whole of said universal cord.

22. An electronic endoscope apparatus according to claim 21, wherein said universal cord cover has one end thereof which is detachably fixed to an operation part and the other end which is detachably fixed to the connector part.

23. An electronic endoscope apparatus according to claim 21, wherein said universal cord cover is grounded.

24. An electronic endoscope apparatus according to claim 21, wherein said universal cord cover has an insulation cover of insulation resin and a metal evaporation layer provided on an inner surface of said insulation cover and having electromagnetic shielding function.

25. An electronic endoscope apparatus according to claim 21, wherein a metal blade having electromagnetic shielding function is provided on the side of an inner peripheral surface of an insulation cover of said universal cord cover.

26. An electronic endoscope apparatus according to claim 1, wherein a shielding curtain is provided as the electromagnetic interference countermeasure member which covers said external devices.

27. An electronic endoscope apparatus according to claim 26, wherein said shielding curtain is so formed that a stainless-steel metal element wire is wound.

28. An electronic endoscope apparatus according to claim 1, wherein an insulation layer is provided on an armor of the image pickup cable which has a coaxial line and/or a simple line and a shielding layer for transmitting an electric signal which is photoelectrically changed by the image pickup element which is provided at a forward end of the insertion part of said endoscope, wherein an electromagnetic interference countermeasure web-like member is further arranged on the outside thereof between a flexible tube part and a connector part, and wherein said electromagnetic interference countermeasure web-like member and the shielding layer of an image pickup cable are non-conductive to each other.

29. An electronic endoscope apparatus according to claim 28, wherein said electromagnetic interference countermeasure web-like member and an armor metal part of the electronic endoscope are conductive to each other, and wherein a shielding layer of the image pickup cable and a video GND of the connector part are conductive to each other.

30. An electronic endoscope apparatus according to claim 1, wherein a signal cable has an end thereof for transmitting, to the video processor, an electric signal which is photoelectrically changed by the image pickup element provided at a forward end of the insertion part of said endoscope, an electric connector being provided at the end of said signal cable separately from the universal cord.

31. An electronic endoscope apparatus according to claim 30, wherein said signal cable is provided within a tube body which has elasticity, and wherein said tube body and said signal cable are detachable with respect to an operation part of said endoscope.

32. An electronic endoscope apparatus according to claim 30, wherein a plurality of electromagnetic absorber members are detachable to said signal cable.

33. An electronic endoscope apparatus according to claim 32, wherein each of said electromagnetic absorber members is a ferrite core which has a hollow part accommodating therein the signal cable, and a cut-out which communicates said hollow part and an outer peripheral face with each other.

34. An electronic endoscope apparatus according to claim 1, further comprising, within the endoscope insertion part of said electronic endoscope,
at least a forceps insertion and passage tube,
a light guide bundle and a light-guide-bundle protection tube for protecting the same, and
an image pickup cable for transmitting an electric signal which photoelectrically converted by the solid-state image pickup element,
wherein said image pickup cable is arranged substantially at a center within the insertion part, and wherein at least the forceps insertion and passage tube and the light-guide-bundle protection tube which consist of a shielding member are arranged around said image pickup cable.

35. An electronic endoscope apparatus according to claim 1, wherein, when lead wires are spirally wound around a signal line which transmits an electric signal which is photoelectrically changed by the image pickup element which is provided at a forward end of said endoscope insertion part, to provide a double shielding layer, the lead wires which form respectively the shielding layers are the same in winding direction as each other.

36. An electronic endoscope apparatus according to claim 35, wherein an insulation layer is provided between the shielding layers which are superposed double one upon the other.

37. An electronic endoscope apparatus according to claim 35, wherein the lead wires which form said double shielding layers are different in diameter size from each other.

38. An electronic endoscope apparatus according to claim 35, wherein diameter sizes of the lead wires which form said double shielding layers are set in conformance with frequency of generated noises.
